(19) **Europäisches Patentamt**
**European Patent Office**
**Office européen des brevets**

(11) **EP 4 487 772 A1**

(12) **DEMANDE DE BREVET EUROPEEN**

(43) Date de publication:
**08.01.2025 Bulletin 2025/02**

(21) Numéro de dépôt: **24186522.9**

(22) Date de dépôt: **04.07.2024**

(51) Classification Internationale des Brevets (IPC):
*A61B 5/145* (2006.01)      *A61B 5/00* (2006.01)

(52) Classification Coopérative des Brevets (CPC):
**A61B 5/685; A61B 5/14514;** A61B 2562/125

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité: **06.07.2023 FR 2307214**

(71) Demandeurs:
• **Commissariat à l'Energie Atomique et aux Energies Alternatives**
**75015 Paris (FR)**

• **Centre National de la Recherche Scientifique**
**75016 Paris (FR)**
• **Université Grenoble Alpes**
**38401 Saint-Martin-d'Hères (FR)**

(72) Inventeurs:
• **GROSS, Andrew**
**38610 GIERES (FR)**
• **DARMAU, Bastien**
**38401 SAINT-MARTIN D'HERES (FR)**
• **TEXIER-NOGUES, Isabelle**
**38054 GRENOBLE CEDEX 09 (FR)**

(74) Mandataire: **Cabinet Nony**
**11 rue Saint-Georges**
**75009 Paris (FR)**

(54) **DISPOSITIF DE TYPE PATCH ÉLECTRODE-MICRO-AIGUILLES**

(57) L'invention a pour objet un dispositif comportant au moins un support polymérique ayant une surface dédiée à un contact avec une peau, au moins un réseau de micro-aiguilles polymériques pleines solidaires dudit support et dépassant vers l'extérieur de ladite surface du support dédiée au contact avec ladite peau et au moins une électrode caractérisé en ce que lesdites micro-aiguilles et au moins la surface de contact dudit support avec ladite peau sont formées d'un hydrogel réticulé, non conducteur électronique à l'état sec et conducteur électrolytique au contact d'un fluide aqueux, et en ce que la, une, des ou lesdites électrodes sont disposées au contact de l'hydrogel dédié à gonfler au contact d'un fluide aqueux et sont dénuées de contact direct avec la peau. L'invention concerne également un procédé de préparation dudit dispositif ainsi que certaines de ses utilisations.

[Fig 1]

Fig. 1

## Description

### Domaine technique

La présente invention concerne le domaine des dispositifs utiles notamment pour la caractérisation et/ou stimulation électrochimique et transdermique de paramètres physiques (pH, conductivité, température) et/ou de biomarqueurs dans les fluides interstitiels. Comme détaillé ci-après, ces dispositifs peuvent également être considérés pour la conversion et/ou le stockage d'énergie, des techniques employant au moins deux électrodes dans une cellule électrochimique avec un électrolyte entre les électrodes, mais également la libération transdermique d'actifs notamment à finalité thérapeutique, technique consistant à faire passer un courant entre 2 électrodes à travers la peau ou entre les tissus sous-cutanés.

En particulier, un tel dispositif se présente sous la forme d'un patch micro-aiguilles, MN (abréviation de « MicroNeedles » en anglais), couplé à au moins une électrode.

### Technique antérieure

L'intérêt des dispositifs pour les analyses transdermiques et plus particulièrement des patchs micro-aiguilles est, qu'une fois insérés dans la peau, la, une, des ou les électrodes qu'ils intègrent peu(ven)t réaliser une mesure d'un analyte ou un travail tel que la contraction d'un muscle sans effort nécessaire.

Toutefois, les méthodes d'intégration entre patch micro-aiguilles et électrodes afin d'aboutir à de tels dispositifs sont aujourd'hui relativement complexes.

Un premier type de dispositif intégré assemble différents patchs micro-aiguilles distincts, chaque patch étant couplé à, ou constituant, une électrode différente à l'image de celui décrit dans [réf 1] Sanjiv Sharma and al. Anal Bioanal Chem, 2016, 408, 8427 - 8435. Dans un autre mode de réalisation, plusieurs patchs dédiés chacun à la caractérisation d'un biomarqueur spécifique sont réunis dans un unique dispositif [Réf 2] Farshad Tehrani and al Nature Biomédical Engineering, 2022. Dans ces exemples, les micro-aiguilles de chaque patch sont traitées en surface externe pour constituer une électrode sur chaque patch, avec un traitement différent pour chaque patch pour constituer des électrodes différentes. On ne peut donc s'affranchir du risque, lors de l'insertion des aiguilles dans la peau, d'une délamination et/ou d'un relargage des matériaux, le plus souvent métalliques non biocompatibles, constituant ces électrodes. Qui plus est, la réalisation des dispositifs combinant plusieurs patchs est compliquée.

Il a également été réalisé des dispositifs avec un unique patch mais dans lequel des micro-aiguilles sont toujours traitées en surface externe différemment pour constituer différents types d'électrodes. Ainsi, le document [Ref 4] US 2006/0264716 décrit un patch de micro-aiguilles en Si / SiO (dopé) dont certaines micro-aiguilles sont recouvertes par de l'Ag / AgCl (« RE ») et d'autres avec des revêtements multicouches, comprenant des particules de Rh, de l'Ag / acétate de cellulose + glucose oxidase, et avec couche protectrice de membrane Nafion ou PTFE pour constituer l'électrode de travail. Les documents [Réf 5] *Hazhir* Teymourian and al. Anal. Chem., 2020, 92, 2, 2291 - 2300 et [Réf 6] AM Vinu Mohan and alBiosensors andBioelectronics, 91, 2017, 574-5791 proposent, selon une autre variante, de placer ces électrodes (de travail/élément de transduction, référence, contre-électrode) à l'intérieur du canal interne d'une micro-aiguille. La micro-aiguille prélève le fluide interstitiel par capillarité et l'amène en contact de l'électrode pour la mesure. Toutefois, ces deux variantes de patch unique requièrent également un procédé de fabrication multi-étapes et complexe et ne permettent pas non plus de s'affranchir du risque de délamination évoqué ci-dessus puisqu'il demeure un risque que du matériau constitutif des électrodes puisse être véhiculé jusqu'à l'intérieur du tissu analysé, via le liquide interstitiel qui est au contact direct avec les électrodes.

Il a été également proposé des patchs micro-aiguilles, comportant en outre un ou plusieurs canaux fluidiques disposés à l'arrière des micro-aiguilles et dans lesquels sont insérées les électrodes [Réf 7] US 20090099427 et [Réf 8] US 20140336487. Néanmoins, demeure encore dans cette variante, le risque de toxicité en cas de relargage du matériau des électrodes, pas toujours biocompatible, dans le fluide interstitiel baignant les tissus. La réalisation d'un canal fluidique complexifie en outre la fabrication de ce type de dispositif. Enfin, la [Réf 3] Peyman G and al. Adv Health Mater. 2022 décrit un dispositif constitué de trois patchs micro-aiguilles polymériques, dont un (celui de l'électrode de travail) à base d'hydrogel gonflant et conducteur, pour obtenir un dispositif de mesure non-enzymatique du glucose. Contrairement à la [Réf 1] et la [Réf 2], l'électrode de travail est cette fois un patch de micro-aiguilles en hydrogel d'acide-hyaluronique-dopamine réticulé, additionné de PEDOT:PSS (PEDOT : Poly(3,4-ethylenedioxythiophene), polymère conducteur, PSS : poly(styrène sulfonate)) et de nanoparticules de Pt et Ag (formées in situ par oxydation de $Ag+$ et $Pt+$ par la dopamine) pour augmenter la conductivité de l'hydrogel. De par la présence du PEDOT:PSS et des nanoparticules Pt et Ag, le matériau n'est pas que conducteur ionique, mais également conducteur électrique et le patch MN constitue une électrode à part entière. Un tel système a donc pour inconvénient de ne pas se prêter à l'intégration d'une contre-électrode, CE, ou d'une électrode de référence, RE, sous peine d'être en court-circuit. En conséquence, il demeure un besoin d'un dispositif notamment utile pour une mesure électrochimique transdermique, couplant au moins un patch micro-aiguilles, MN, avec une ou plusieurs électrodes, et qui soit dénué de risque de toxicité à l'égard du sujet faisant l'objet de cette mesure.

Il demeure également le besoin qu'un tel dispositif soit compatible avec la mise en oeuvre de différentes combinaisons d'électrodes telles que par exemple électrodes de travail, WE, de référence, RE, et contre-électrode, CE, et qui à ce titre ne soit pas lui-même conducteur électrique par opposition notamment à un patch formé d'un hydrogel conducteur PEDOT :PSS tel que décrit en [Réf 3].

Il demeure également un besoin qu'un tel dispositif soit de fabrication non complexe.

La présente invention vise précisément à répondre à ces besoins.

**Résumé de l'invention**

Ainsi, selon un de ses aspects, la présente invention concerne un dispositif comportant au moins un support polymérique 1 ayant une surface 4 dédiée à un contact avec une peau 2, au moins un réseau de micro-aiguilles polymériques pleines 3 solidaires dudit support 4 et dépassant vers l'extérieur de ladite surface 4 du support 1 dédiée au contact avec ladite peau 2, et au moins une électrode 9 caractérisé en ce que lesdites micro-aiguilles 3 et au moins la surface de contact 4 dudit support 1 avec ladite peau 2 sont formées d'un hydrogel réticulé, biocompatible, non conducteur électronique à l'état sec et conducteur électrolytique au contact d'un fluide aqueux, et en ce que la, une, des ou lesdites électrodes 9 sont disposées au contact de l'hydrogel dédié à gonfler au contact dudit fluide aqueux et sont dénuées de contact direct avec la peau 2.

De manière inattendue, les inventeurs ont ainsi constaté que le couplage d'électrode(s) à un support comprenant au moins un réseau de micro-aiguilles peut être réalisé dans des conditions permettant de satisfaire aux attentes précitées sous réserve que ce substrat soit notamment formé d'un hydrogel conforme à l'invention, à savoir apte à évoluer d'un état non conducteur à l'égard des électrons lorsqu'il est à l'état sec vers un état conducteur électrolytique lorsqu'il est gonflé par un fluide aqueux.

Plus précisément, et comme il ressort des exemples ci-après, les micro-aiguilles 3 du dispositif selon l'invention possèdent avantageusement à l'état sec, la résistance mécanique nécessaire à la perforation d'un tissu corporel, notamment à des profondeurs comprises entre 200 et 2000 $\mu$m dans le cas d'une peau humaine, pour accéder à son fluide interstitiel, ISF, de façon efficace. Lorsque l'hydrogel réticulé et sec, constitutif de ces micro-aiguilles 3, se trouve au contact de ce fluide ISF, il se gonfle et le fluide à analyser va y diffuser par différence de pression osmotique et par capillarité. Ainsi, d'un point de vue électrique, les micro-aiguilles 3, de même que la partie du support 1 solidaire des micro-aiguilles 3, peuvent être considérées, lorsqu'elles sont imprégnées d'ISF, comme devenant une prolongation des tissus avec l'ISF et assurent alors une fonction d'électrolyte. L'électrode ou les électrode(s) 9 disposée(s) au contact de cet hydrogel gonflé d'ISF peu(ven)t alors réaliser une mesure ou opération fidèle à celle qui serait réalisée si elle(s) étai(en)t insérée(s) directement dans la peau 2 mais avantageusement de façon minimalement invasive.

Selon une variante de réalisation, la, une, des ou les électrodes 9 est (sont) intégrée(s) dans l'hydrogel réticulé constitutif du support 1 qui est en contact direct avec l'arrière des micro-aiguilles 3.

Selon une autre variante de réalisation, la, une, des ou les électrodes 9 sont intégrées dans l'hydrogel réticulé constitutif de microaiguille(s) 3. En particulier, elles y sont disposées de manière à n'y avoir un contact qu'avec l'ISF qui y diffuse par capillarité et/ou différence de pression osmotique. Ainsi, par opposition à d'autres dispositifs de l'art antérieur, les électrodes 9 ainsi disposées du dispositif de l'invention n'ont pas de contact direct avec la peau 2 faisant l'objet de l'analyse.

Selon encore une autre variante de réalisation, le dispositif selon l'invention intègre au moins une électrode dans l'hydrogel réticulé constitutif du support 1 en contact direct avec l'arrière des micro-aiguilles 3 et au moins une électrode dans l'hydrogel réticulé constitutif de micro-aiguilles 3.

Le dispositif selon l'invention est donc avantageux à plusieurs titres.

Son hydrogel réticulé constitutif est compatible avec l'intégration de n'importe quelle électrode (poreuse ou non) pour la détection des analytes, ions et biomarqueurs, en particulier une bioélectrode avec un revêtement enzymatique sur l'électrode. Cet hydrogel fournit ainsi à la surface et éventuellement en profondeur de la/des électrode(s) un environnement aqueux approprié pour fonctionner dans des conditions optimales et prolonger leur durée de vie/stabilité et donc la durée de vie/stabilité du dispositif transdermique.

Seul son composant hydrogel réticulé est en contact direct avec le tissu biologique faisant l'objet de l'analyse, et donc le risque de délaminage du revêtement et/ou matériau constitutif de l'électrode (par exemple, médiateur, enzyme, couche AgCl, etc...), lors de l'insertion des micro-aiguilles dans le tissu est écarté.

Le risque de détachement de composant(s) d'une électrode est également écarté compte-tenu de l'intégration de celle-ci au sein de l'hydrogel réticulé du dispositif selon l'invention. De même, la chute de tension (ou chute ohmique) causée par la résistance de la solution entre des électrodes de travail et de référence peut y être minimisée grâce à une intégration intime dans le même patch de micro-aiguilles.

Le positionnement de l'électrode près de l'arrière des pointes des micro-aiguilles et/ou dans des micro-aiguilles elles-mêmes, limite également la distance que l'analyte, ion, proton, biomarqueur doit parcourir et/ou toute limitation de transport de masse qui pourrait affecter les performances du capteur, y compris la réduction du temps de latence.

Selon une variante préférée, l'ensemble support 1 et micro-aiguilles 3 est constitué dudit hydrogel réticulé, biocompatible, non conducteur électronique à l'état sec et gonflant au contact d'un fluide aqueux.

En particulier, l'hydrogel réticulé est dénué de constituant métallique et de polymère conducteur électronique.

En particulier, le dispositif selon l'invention se présente sous la forme d'un patch micro-aiguilles, MN.

Selon un autre de ses aspects, la présente invention concerne un procédé de préparation d'un dispositif selon l'invention, notamment par micromoulage, et comprenant au moins la réticulation d'au moins un polymère, de préférence un biopolymère, pour former ledit hydrogel réticulé caractérisé en ce que la ou les électrodes 9 dudit dispositif sont intégrées par mise en contact avec ledit polymère avant ou simultanément à sa réticulation.

En particulier, la ou les électrodes 9 sont disposées au contact de la formulation aqueuse contenant au moins ledit polymère non réticulé et y subissent une pression mécanique pour les positionner en profondeur à proximité de la base des micro-aiguilles 3 et/ou en profondeur de micro-aiguille(s) 3, préalablement ou simultanément à la réticulation.

Selon encore un autre de ses aspects, la présente invention concerne l'utilisation d'un dispositif selon l'invention pour la conversion d'énergie, pour le stockage d'énergie ou pour des mesures électrochimiques transdermiques, en particulier pour la caractérisation d'au moins un analyte dans un liquide interstitiel 6.

Ainsi, l'invention vise tout particulièrement, un procédé de détection et/ou de dosage d'au moins un analyte dans un liquide interstitiel 6 d'un patient utilisateur, comprenant au moins la mise en contact des micro-aiguilles 3 d'un dispositif selon l'invention avec un liquide interstitiel 6 dudit patient dans des conditions propices au gonflement dudit hydrogel réticulé constitutif des micro-aiguilles 3 par ce liquide 6 et à la diffusion de ce liquide 6 par différence de pression osmotique et/ou capillarité jusqu'à la ou les électrode(s) 9 dudit dispositif. D'autres caractéristiques, variantes et avantages des objets de l'invention ressortiront mieux à la lecture de la description, des exemples et figures qui vont suivre, donnés à titre illustratif et non limitatif de l'invention.

Dans la suite du texte, les expressions « compris entre ... et ... », « allant de ... à ... » et « variant de ... à ... » sont équivalentes et entendent signifier que les bornes sont incluses, sauf mention contraire.

## Brève description des dessins

[Fig 1] représente un exemple de schéma de dispositif selon l'invention.

[Fig 2a] représente le chronoampérogramme d'une électrode de travail WE intégrée au sein d'un patch Dex-MA MNs (exemple 2) montrant l'évolution du courant après ajouts successifs de glucose à des temps réguliers à -0,1V vs pseudo référence Ag/AgCl

[Fig 2b] représente la courbe d'étalonnage de la même électrode de travail WE qu'à la figure précédente intégrée au sein d'un patch Dex-MA MNs (exemple 2) intégrée au sein du même patch MN.

[Fig 3] rend compte des résultats obtenus à l'issu des essais de cytotoxicité détaillés en exemple 5.

## Description détaillée

Comme il ressort de ce qui précède, l'invention vise à proposer des dispositifs peu invasifs pouvant intervenir transdermiquement, en continu ou non, pour notamment la caractérisation et/ou stimulation de paramètres physiques (pH, conductivité, température), et/ou la détection transdermique de biomarqueurs dans les fluides interstitiels.

Plus particulièrement, ce dispositif comprend au moins un support polymérique 1, au moins une électrode 9 et des micro-aiguilles 3 disposées sur l'une des faces du support. Les micro-aiguilles 3 pénètrent dans un tissu biologique jusqu'au liquide interstitiel 6 de celui-ci et dans lequel il est précisément cherché à caractériser électrochimiquement au moins un paramètre physique et/ou une espèce chimique ou biologique ou à effectuer une stimulation électrique par exemple.

Dans le dispositif selon l'invention, les micro-aiguilles 3 sont pleines. En d'autres termes, elles ne sont pas creuses mais constituées d'hydrogel réticulé conforme à l'invention et, le cas échéant, d'une électrode 9 intégrée dans cet hydrogel.

Les micro-aiguilles 3 ont de préférence une longueur variant de 200 à 3000 $\mu$m.

Dans une variante de réalisation, le dispositif selon l'invention peut comprendre plusieurs réseaux de micro-aiguilles 3 et/ou plusieurs électrodes 9.

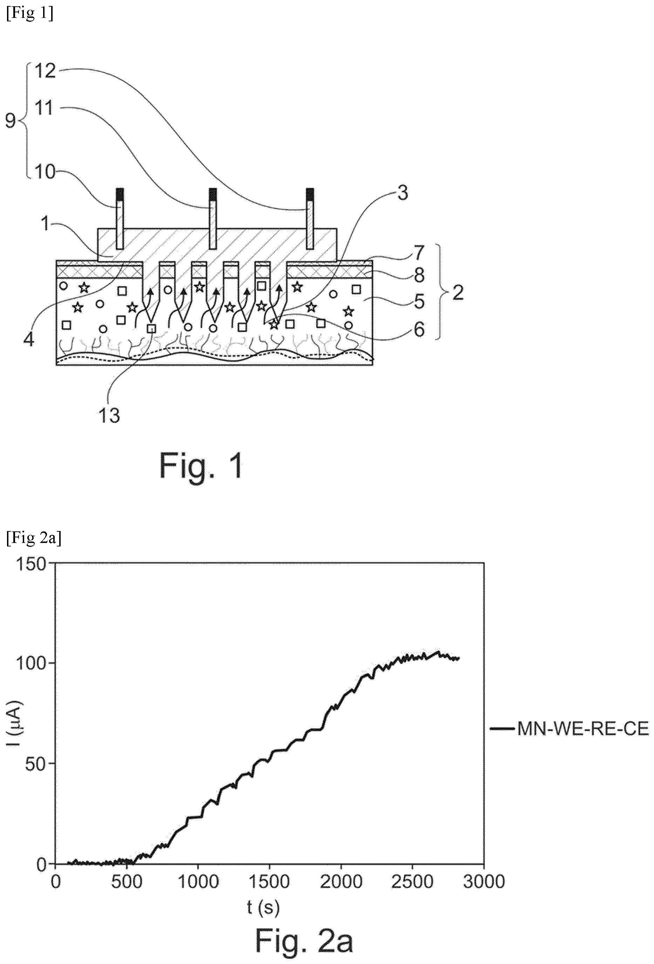

En particulier, le dispositif selon l'invention consiste en un patch micro-aiguilles, MN, notamment tel qu'illustré en Fig 1.

Le dispositif en Fig 1 est formé d'un corps fait d'un hydrogel réticulé qui est, avant utilisation, à l'état sec. Le corps comporte un support 1 et des micro-aiguilles 3 qui font saillie d'une surface 4 du support 1 en contact avec la peau 2 d'un patient et qui ont pénétré dans le derme 5 de la peau du patient, à travers la couche cornée 7, et l'épiderme 8.

Selon le dispositif de la Fig 1, le dispositif comporte une électrode de référence 10 pour définir un potentiel électrique de référence, une électrode de travail 11 et une contre-électrode 12 qui définit un circuit de circulation électrique avec l'électrode de travail 11 de façon à maintenir une tension constante dans l'électrode de référence 10.

L'électrode de référence 10, l'électrode de travail 11, et la contre-électrode 12 sont intégrées dans le volume du support 1.

Le fluide interstitiel 6 présent dans le derme 5 comprenant les biomarqueurs 13 diffuse vers les électrodes 10, 11, 12 à travers les micro-aiguilles 3 puis dans le support 1, en formant un hydrogel réticulé gorgé d'électrolyte (fluide interstitiel).

Le raccordement électrique, non représenté en Fig 1, peut être formé par un fil en un matériau électriquement conducteur (par exemple un fil de cuivre argenté) adhérant au dos de l'électrode considérée avec une pâte conductrice (tel que de la pâte de carbone).

<u>Hydrogel réticulé</u>

Au sens de l'invention, un hydrogel réticulé est un polymère tridimensionnel hydrophile et insoluble. Sa réticulation est obtenue par des liaisons physiques et/ou chimiques, de préférence chimiques, existantes entre des chaînes polymériques.

A l'état sec, l'hydrogel réticulé possède la dureté nécessaire à la pénétration des micro-aiguilles 3 qu'il compose mais, en revanche, n'est pas apte à assurer la circulation d'électrons lui-même. En d'autres termes, il n'est pas conducteur ou semi-conducteur électronique. Il est ainsi dénué de constituant métallique et de polymère conducteur organique à l'image par exemple du PEDOT, du poly(pyrrole) ou de la poly(aniline).

Par ailleurs, cet hydrogel réticulé et sec est capable de gonfler au contact d'un fluide, notamment un fluide aqueux comme de l'eau ou un fluide biologique. Il peut donc absorber et retenir une grande quantité d'un fluide. Ce taux de gonflement de l'hydrogel (SR) est généralement défini par le rapport entre le poids de l'hydrogel gonflé et le poids de ce même hydrogel à l'état sec correspondant. Plus précisément, le fluide diffuse à l'intérieur de l'hydrogel réticulé par des phénomènes de capillarité et/ou de différence de pression osmotique entre le fluide et l'hydrogel. Ainsi imprégné, l'hydrogel réticulé rend possible la mise en contact du fluide aqueux à caractériser avec notamment l'électrode de travail qu'il intègre. En d'autres termes, le gonflement de l'hydrogel par le liquide interstitiel 6 fait que le dispositif selon l'invention, isolant à l'état sec, devient une matrice électrolytique (conducteur ionique), et permet de faire un pont électrolytique entre le tissu où sont insérées les micro-aiguilles 3 et la ou les électrodes 9 qu'il contient. Par opposition à des dispositifs de l'art antérieur, l'hydrogel réticulé et gonflé selon l'invention agit en outre comme un filtre isolant la ou les électrodes 9, qu'il intègre, de tout contact possible avec le tissu biologique. Le réseau structural du gel réticulé est tel, qu'à l'état gonflé, il rend quasi impossible toute migration d'éventuels petits fragments d'électrode vers ce tissu. Tout risque de toxicité du fait des électrodes 9 est donc exclu ou fortement minimisé.

L'hydrogel réticulé selon l'invention est également biocompatible.

Au sens de l'invention, un hydrogel biocompatible est un hydrogel qui, du fait de sa nature chimique, n'est pas de nature à provoquer un effet indésirable, comme de l'inflammation, au niveau du tissu biologique faisant l'objet de l'analyse.

L'aspect biocompatible de l'hydrogel est un atout car seul lui est en contact direct avec les tissus en tant que composant des micro-aiguilles 3 pénétrant dans la peau 2 d'un sujet. Ainsi, l'hydrogel constitutif des micro-aiguilles 3 et du support 1 joue le rôle d'écran entre la peau 2 et la ou les électrodes 9 qui lui sont associées dans le dispositif selon l'invention. De plus, si l'hydrogel bien que réticulé reste biorésorbable sur le long terme, les micro-aiguilles 3 en polymère ne présenteront aucun risque en cas de casse dans la peau 2 pendant l'utilisation du dispositif, puisque leur composant pourra être résorbé au cours du temps sans dommage pour les tissus.

Enfin, compte-tenu de sa biocompatibilité, l'hydrogel peut jouer un rôle bénéfique à l'égard de la stabilité de bioélectrode(s) 9 susceptible(s) de lui être associée(s) dans le dispositif selon l'invention. Il constitue un environnement biocompatible pour les enzymes, peut réduire leur dénaturation et peut permettre un transport de masse favorable.

Comme déjà précisé, l'hydrogel réticulé selon l'invention possède avantageusement de bonnes propriétés de gonflement (gonflement en 24h supérieur à 10%, de préférence supérieur à 20%, plus préférentiellement plus de 50%) et à l'état sec une bonne dureté mécanique pour transpercer la peau (module d'Young supérieur à 10 kPa).

L'hydrogel réticulé selon l'invention peut être obtenu par réticulation de tout type de polymère qui, une fois réticulé, forme, en contact avec un fluide aqueux, un hydrogel insoluble et infusible.

En particulier, l'hydrogel réticulé selon l'invention peut être obtenu par réticulation d'un ou plusieurs polymères synthétiques, d'un ou plusieurs biopolymères, le cas échéant modifiés chimiquement pour être réticulables, ou d'un de leurs mélanges.

Au sens de l'invention, le terme "biopolymère" désigne un polymère dérivant de ressources naturelles renouvelables (un matériau dit encore « naturel »). Les biopolymères considérés selon l'invention ont l'avantage de posséder, outre une très bonne compatibilité avec la peau humaine, une biodégradabilité en tant que tels.

Ce terme biopolymère couvre ainsi les polymères naturels tels que par exemple une protéine ou un polysaccharide mais également les dérivés de polymères naturels comme par exemple ceux qui dérivent d'une modification chimique de ces polymères naturels pour les rendre réticulables ou compatibles avec un mode de réticulation spécifique.

Cette modification chimique consiste le plus souvent à introduire dans la structure des polymères à modifier, des motifs réticulables en particulier choisis parmi les motifs thiol, divinylsulfonyle, maléimide, azoture, alcynyle, alcènyle, acrylate, méthacrylate, aldéhyde, norbornènyle, oxy-amine et leurs mélanges, et en particulier parmi les acrylates, méthacrylates et leurs mélanges.

A titre illustratif de polymères synthétiques convenant pour former un hydrogel réticulé convenant à l'invention, peuvent notamment être cités les poly(éthylène glycol) (PEG) brut ou modifiés par différentes fonctions chimiques comme par exemple les poly(éthylène glycol) diacrylique (PEGDA), poly(éthylène glycol)diméthacrylique (PEGDMA) les poly(vinyl pyrrolidone) (PVP), les poly(méthyl vinyl éther/acide maléique-PEG, les PVA-MA, -PLA, -PLLA, -PGA, -PLGA et leurs dérivés.

A titre illustratif de biopolymères convenant pour former un hydrogel réticulé conforme à l'invention, peuvent notamment être cités les polyhydroxyacides, tels que par exemple l'acide polyglycolique (PGA) et l'acide polylactique (PLA), également connu sous le nom de polylactide ; les polysaccharides, tels que par exemple la cellulose, la chitine et l'amidon et des protéines ou des peptides, tels que par exemple le collagène, la gélatine, les élastines, les fibroïnes, la zéine de maïs, la soie de diverses espèces de vers à soie, la kératine et leurs dérivés.

Selon une variante de réalisation préférée, l'hydrogel réticulé et biocompatible considéré selon l'invention est obtenu par réticulation d'au moins un biopolymère choisi parmi les polymères de type chitosane, dextran, alginates, collagène, gélatine, agarose, chitine, polyhydroxyalcanoates, pullane, amidon, amylose, amylopectine, cellulosique en particulier carboxyméthylcellulose, et hydroxypropylcellulose, acide hyaluronique, gomme gellane, gomme xanthane comme par exemple les dérivés de ces polymères issus de leur modification chimique pour les rendre réticulables ou compatibles pour un mode de réticulation spécifique et leurs combinaisons.

Selon un mode de réalisation particulier, ce biopolymère est, ou dérive, d'au moins un polymère choisi parmi les alginates, l'acide hyaluronique, la carboxyméthylcellulose, le chitosan, le dextran et leurs dérivés, et, de préférence, le dextran.

En particulier, l'hydrogel est un hydrogel réticulé de dextran.

Au sens de l'invention, le terme dextran couvre les dextrans ayant été ou non chimiquement modifiés pour être réticulés.

Ainsi, il peut être intéressant de réticuler un polymère de dextran chimiquement modifié par des motifs acrylates et/ou méthacrylates qui sont bien connus pour être photopolymérisables. Ces groupements fonctionnels qui rendent possible une réticulation par irradiation, sont introduits avec un degré de substitution donné, DS. Conventionnellement, le DS correspond au nombre de fonction polymérisable introduit sur 100 unités monomères. Ce DS est déterminable par $^1$H RMN. Dans ce qui suit ; il est exprimé en pourcentage.

Ainsi, selon un mode de réalisation particulier, ledit hydrogel dérivant de la réticulation d'au moins un polymère de dextran modifié par des motifs choisis parmi les acrylate, méthacrylate, alcényle, et alcynyle, de préférence un polymère de dextran modifié par des motifs méthacrylate, DexMA, et de DS variant de 5 à 65 %.

Electrodes et leur mode d'intégration

Une autre spécificité du dispositif de l'invention repose sur le mode d'intégration des électrodes.

Comme précisé ci-dessus, l'électrode ou les électrodes 9 sont disposées de manière à ne pas avoir de contact direct avec le tissu dont l'ISF 6 fait l'objet de l'analyse.

D'une manière générale, elles sont entièrement intégrées dans l'hydrogel réticulé devant être gonflé par cet ISF 6, à l'exception de leur extrémité dédiée au raccordement électrique externe. Généralement, ce raccordement est localisé sur la face supérieure ou l'une des faces latérales du dispositif.

Par opposition à des dispositifs classiques, l'électrode ou les électrodes 9 ne sont également pas, dans le cadre de la présente invention, en communication avec un canal auquel seraient reliées les micro-aiguilles 3.

Comme déjà précisé ci-dessus, elles sont en revanche disposées soit à proximité et/ou au sein de micro-aiguilles pleines 3 du dispositif.

Selon un mode de réalisation particulier, elles sont intégrées dans l'épaisseur du support 1 localisé à l'arrière des micro-aiguilles.

Ce mode d'intégration est notamment illustré en Fig 1.

Comme précisé ci-dessus, le dispositif est compatible avec la mise en oeuvre de plusieurs électrodes 9 dans la mesure où l'hydrogel réticulé le constituant est non conducteur électronique. Ces électrodes 9 peuvent être choisies parmi des électrodes de référence, contre-électrodes, électrodes de travail telles qu'électrodes sélectives aux ions (ISE), électrodes de pH, électrodes électrocatalytique, bioélectrodes, électrodes micro ou nanostructurées.

Ainsi, le dispositif peut comprendre une ou plusieurs électrodes 9 choisies parmi les électrodes ou bioélectrodes à base de Pt, Au, Ag, Pd, Ni, Ir, carbone graphitique, carbone amorphe, graphène, oxyde de graphène, diamant, diamant dopé au bore, nanotubes, fibres dopées semi-conductrices, par exemple et de préférence silicium dopé, d'oxydes métalliques, par exemple et de préférence oxyde d'indium-étain, et les électrodes en polymères conducteurs tels que notamment et de préférence PEDOT et fibres conductrices. En fait, tout type d'électrode et/ou de capteur électrochimique de manière générale (capteur, biocapteur, capteur photo-électrochimique avec électrode transparente dans le visible), peut être intégré dans un dispositif selon l'invention dès que le capteur considéré nécessite la diffusion d'un milieu aqueux électrolytique jusqu'au capteur.

L'électrode 9 peut être une macro, micro ou nanoélectrode.

La forme générale de l'électrode 9 peut être plane ou non. Les électrodes 9 peuvent comprendre une feuille de papier carbone ou de tissu ou une feuille ou une pastille ou une électrode imprimée, par ex. électrode sérigraphiée. Les électrodes de formes non planes comprennent un fil de Pt ou un fil de fibre de carbone ou une électrode enroulée.

Par exemple, ces électrodes 9 peuvent être choisies parmi les électrodes ou bioélectrodes à base de Pt, Au, Ag, Pd, Ni, Ir, carbone graphitique, carbone amorphe, graphène, oxyde de graphène, diamant, diamant dopé au bore, nanotubes, fibres dopées semi-conductrices ex. silicium dopé, oxydes métalliques, par ex. oxyde d'indium-étain, électrodes polymères conductrices e.g. PEDOT et des fibres conductrices.

Selon une première variante, le dispositif comprend une unique électrode 9 et en particulier une électrode de travail. Le terme « électrode de travail » ici désigne une électrode à laquelle un composé candidat (analyte, biomolécule, agent actif) est électro-oxydé ou électro-réduit avec ou sans l'intervention d'un médiateur redox. Le terme « médiateur redox » désigne un agent de transfert d'électrons qui transfère des électrons entre un composé et une électrode de manière directe ou indirecte. Un médiateur redox est souvent incorporé aux WE de type bioélectrode (électrode à enzyme ; électrode enzymatique) pour faciliter un transfert des électrons entre une enzyme (oxydoréductase) et une électrode pour permettre l'oxydation ou réduction d'un composé candidat. La WE de type (bio)électrode peut notamment jouer le rôle de capteur (bio)électrochimique. De tels capteurs sont généralement couplés en solution avec une contre-électrode et une électrode de référence. Le capteur fonctionne de sorte qu'un courant est généré entre l'électrode de travail et la contre-électrode. De telles électrodes sont particulièrement intéressantes pour détecter et/ou doser un analyte chimique ou biologique comme par exemple des protéines, des acides aminés, virus, hormones, des médicaments, et des drogues et en particulier un analyte choisi parmi les glucose, lactate, alcool, nitrate, alanine, cystéine, pyruvate, glycérol, ions Ca2+, Mg2+, K+, phosphate, urée, cholestérol, glutamate, peroxyde d'hydrogène, hydrogène, etc. L'électrode de travail est souvent fabriquée à base de métal ou carbone et est utilisée pour l'électro-oxydation ou électro-réduction du composé candidat et souvent avec un ou plusieurs éléments catalytiques ou électrocatalytiques comme des métaux nobles, des nanoparticules,des molécules organométalliques, et des enzymes. Il peut en particulier s'agir d'électrodes de travail avec des éléments de reconnaissance (souvent dites 'récepteurs') pour faciliter la détection spécifique et sélective et souvent avec un élément polymérique pour faciliter des propriétés comme les propriétés mécaniques, de reconnaissance et/ou de transmission de signal.

Il est également envisageable de combiner plusieurs électrodes de travail respectivement dédiées à caractériser des analytes chimiques ou biologiques et/ou paramètres physiques différents.

Selon une seconde variante, le dispositif peut comprendre une contre-électrode, CE. De telles électrodes sont particulièrement intéressantes pour établir un circuit avec une électrode de travail sur lequel le courant est appliqué ou mesuré. Les électrodes de Pt, Ag, AgCl, ou autres métaux (Ti, Au, Pd, étain), oxydes métalliques ($IrO_2$), électrodes de carbone (carbone vitreux), électrodes en polymères conducteurs (PEDOT), sont des électrodes auxiliaires typiquement utilisés en électrochimie.

Selon autre variante, le dispositif peut comprendre au moins une électrode de référence, RE. De telles électrodes sont particulièrement intéressantes pour la mesure ou le contrôle du potentiel d'un indicateur ou de l'électrode de travail. Les électrodes au calomel saturé ou électrode au chlorure d'argent sont des électrodes de référence typiquement utilisées en électrochimie.

Selon encore une autre variante, le dispositif comporte au moins deux, voire au moins trois, voire quatre électrodes 9. Un dispositif selon l'invention peut ainsi intégrer par exemple, une à cinq électrodes de travail pour la détection d'un à cinq analytes différents avec, le cas échéant, une électrode de référence et une contre-électrode (ou électrode hybride RE/CE). Il peut notamment s'agir de capteurs (bio)chimiques : potentiométriques ; ampérométriques; coulométriques; impédimétriques et/ou bipotentiostatiques.

Un dispositif selon l'invention peut également intégrer plusieurs capteurs choisis parmi les capteurs physiques (ex. conductivité de la solution) et des actionneurs pour l'électrostimulation et l'administration de médicaments (électrophorèse, électrosmose), l' électrosynthèse, l'électrorégénération,

Un dispositif selon l'invention peut ainsi intégrer deux électrodes 9 de charge opposée pour une mesure ou activation physique (e.g. une décharge de courant).

Un dispositif selon l'invention peut aussi intégrer deux électrodes électrocatalytiques de charge opposée (e.g. anode et cathode) 9 pour la production ou le stockage d'énergie. Bien entendu, un dispositif selon l'invention et dédié à une mesure ou activation physique peut intégrer également une unique électrode 9.

Le dispositif selon l'invention peut également comporter un dispositif de détection électrochimique, attaché à ladite ou auxdites électrodes 9 via des connections électriques, préférentiellement entourées d'isolant. Ce dispositif de détection permet ainsi de réaliser des mesures par potentiométrie, voltamétrie cyclique (CV), voltamétrie cyclique à balayage rapide (FSCV), voltamétrie à onde carrée (SWV), voltampérométrie pulsée ou chronoampérométrie.

Procédé de préparation d'un dispositif selon l'invention

Comme précisé ci-dessus, l'invention vise également un procédé de préparation d'un dispositif selon l'invention comprenant au moins la réticulation d'au moins un polymère, en particulier un biopolymère, pour former ledit hydrogel réticulé caractérisé en ce que la ou les électrodes 9 dudit dispositif sont intégrées par mise en contact avec ledit polymère avant ou simultanément à sa réticulation.

En particulier, lors de la mise en contact de la ou des électrodes 9, le polymère est mis en oeuvre sous la forme d'une formulation aqueuse, liquide ou semi-liquide, c'est-à-dire d'aspect visqueux et, de préférence, présentant une viscosité variant de 1 à 100 Pa.s$^{-1}$ à 1 Hz lorsque la une contrainte de cisaillement appliquée est de 0,2 %. La solution de polymère est préférentiellement préparée en solution aqueuse, avec ou sans sels, comme par exemple NaCl, KCl, LiCl, ou avec ou sans tampon, comme par exemple un tampon phosphate. Cette formulation liquide, de préférence aqueuse, contient ledit polymère et, de préférence, au moins un agent réticulant.

La ou les électrodes 9 à intégrer sont disposées en surface de cette formulation aqueuse contenant au moins ledit polymère non réticulé et y subissent une pression mécanique pour les positionner en profondeur à proximité de la base des micro-aiguilles 3 et/ou au sein de l'hydrogel constitutif de micro-aiguilles 3, préalablement ou simultanément à la réticulation. En effet, à cette viscosité, la/les électrode(s) 9 avec leur prise de contact peu(ven)t être insérée(s) aisément avec une force modérée (pression légère du pouce par exemple) de façon précise et/ou reproductible. Elles peuvent être insérées de façon que les fils de reprise de contact (ou d'autres contacts électriques) sortent préférentiellement du dispositif par le haut et/ou le côté du support 1 solidaire des micro-aiguilles 3.

Après insertion de la/les électrode(s) 9, la réticulation est réalisée ou poursuivie jusqu'à l'obtention d'un hydrogel réticulé et sec. Pour ce faire, l'opération de réticulation est généralement suivie d'une opération de séchage.

La réticulation peut être une réticulation chimique. En tel cas, un réticulant est également présent dans la formulation liquide contenant le ou les polymère(s) et de préférence le ou les biopolymère(s) à réticuler. Ce mode de réticulation souvent lent est propice à l'intégration de la ou des électrodes 9 au cours de la réticulation.

La réticulation peut être également déclenchée à l'aide d'un stimulus physique comme notamment la chaleur, une exposition gazeuse ou une irradiation (par exemple, lumière, rayonnement UV, rayons X, rayons gamma, rayonnement micro-ondes).

Selon un mode de réalisation particulier, la réticulation est réalisée par voie photochimique. D'une manière avantageuse, les dispositifs selon l'invention peuvent être préparés par une technique de micromoulage utilisant un moule en silicone, lui-même obtenu à partir d'un modèle maître réalisé par micro-usinage.

La formulation liquide, de préférence aqueuse, contenant le biopolymère et, de préférence, au moins un agent réticulant est versé dans le moule obtenu à partir de ce modèle maître. Ce moule permet d'obtenir après évaporation de l'eau ou du solvant de la solution, et réticulation, le dispositif en hydrogel réticulé sec qui peut être démoulé.

Utilisation

Selon une première variante, un dispositif selon l'invention peut être utilisé pour la stimulation électrique. La stimulation électrique transdermique est une technique consistant souvent à envoyer une impulsion électrique à travers la peau afin de réaliser un travail tel que la contraction d'un muscle sans effort nécessaire. Cette technique peut notamment permettre de traiter des maladies comme des douleurs chroniques et est très utilisée dans le domaine médical. Dans des cas particuliers, elle permet de faciliter le passage de molécules à travers le derme pour des applications de délivrance d'espèces tels que des principes actifs, vaccins, etc... Cette technique consiste à faire passer un courant entre deux électrodes à travers la peau. Alternativement, la stimulation électrochimique peut se faire entièrement en passant un courant entre deux électrodes dans les tissus sous-cutanés. L'utilisation d'un dispositif transdermique permet également de stimuler des couches plus en profondeur de la peau par rapport aux systèmes conventionnels.

Selon une seconde variante, un dispositif selon l'invention peut être utilisé pour la génération d'énergie. Il peut par exemple être mis en oeuvre à titre d'une pile enzymatique à biocombustible (ou simplement biopiles). Ce type de pile utilise des enzymes pour produire de la puissance électrique dans des milieux complexes, par exemple, dans le fluide interstitiel (ISF). Le principe de la biopile transdermique est basé sur l'intégration dans le liquide subdermique de deux électrodes assurant le fonctionnement du générateur : une électrode dites « anode » avec une ou plusieurs enzymes pour catalyser l'électrooxydation du ou des combustibles (e.g. glucose) et une électrode dite « cathode » pour l'électroréduction de l'oxydant, comme l'oxygène. Les deux électrodes sont spatialement séparées dans le même fluide. La cathode dans une biopile comprend souvent une enzyme comme catalyseur pour cette réaction d'électro-reduction. Une propriété particulière et surtout pratique pour l'application dans une solution in vivo à titre de biopile enzymatique est que la sélectivité des enzymes immobilisées sur l'électrode permet un design de cellule électrochimique sans besoin d'une membrane séparatrice spécifique (par exemple, une membrane échangeuse de protons). Une biopile comprend, en outre, des moyens de mise en circuit électrique de ladite biopile avec un récepteur électrique. D'une manière générale, une biopile transdermique peut fournir une solution d'énergie autonome pour alimenter des dispositifs avec un besoin d'énergie électrique ponctuel comme des dispositifs électroniques de type capteur ou stimulateur.

Selon une autre variante, un dispositif selon l'invention peut être utilisé pour le stockage d'énergie. Il peut par exemple être mis en oeuvre à titre d'un supercondensateur (par exemple avec une structure à double couche électrochimique ou « Electrochemical Double Layer Capacitor » en anglais) ou d'un dispositif hybride de pile enzymatique à biocombustible-supercondensateur. Ce type de condensateur comprend deux électrodes, souvent en carbone, par exemple du charbon actif ou des nanotubes de carbone. Ces électrodes sont imprégnées d'un électrolyte et spatialement séparées, par exemple, par une membrane isolante et poreuse, de façon à permettre la conduction ionique. Il se forme une zone de charges d'espace (appelée double couche électrique) à chaque interface électrode-électrolyte. D'une manière générale, un supercondensateur peut stocker de l'énergie et en fournir rapidement pour alimenter divers dispositifs électroniques.

Dans une autre variante, le supercondensateur peut être rechargé par conversion en électricité par une (bio)pile sans devoir être connecté à une source d'énergie électrique externe.

Un dispositif selon l'invention est avantageusement utile pour des mesures électrochimiques transdermiques, en particulier pour la caractérisation d'au moins un analyte dans un liquide interstitiel 6.

Au sens de l'invention, le terme analyte couvre toute espèce chimique ou biologique comme un médicament, un agent bioactif, un métabolite, un biomarqueur ou encore un produit biochimique endogène.

Les exemples incluent, mais sans s'y limiter, le glucose, le sodium, le potassium, l'alcool, le lactate (important pour les athlètes), le cortisol, l'urée, les drogues (par exemple, les cannabinoïdes, les amphétamines, la cocaïne et les opioïdes) et les métabolites de la nicotine (par exemple, la cotinine) ainsi que les substances médicamenteuses qu'un patient peut prendre pour une ou plusieurs conditions médicales.

Ainsi, la présente invention vise également un procédé de détection et/ou de dosage d'au moins un analyte 13 dans un liquide interstitiel 6, notamment d'une manière non invasive, comprenant au moins la mise en contact des micro-aiguilles 3 d'un dispositif selon l'invention, avec ledit liquide interstitiel 6 dans des conditions propices au gonflement dudit hydrogel constitutif des micro-aiguilles 3 par ce liquide et à la diffusion de ce liquide par différence de pression osmotique et/ou capillarité jusqu'à la ou une des électrode(s) 9 dudit dispositif.

Les exemples et figures qui suivent sont présentés à titre illustratif et non limitatif du domaine de l'invention.

**Matériels**

Dextran T70 (Dex T70, Mw = 70 000 g/mol) et Dextran T20 (Dex T20, Mw = 20 000 g/mol) de Pharmacosmos,

Gélatine de peau de porc (gel strength 300, Type A)
Sérum albumine bovine (BSA, $\geq$ 95%) de Sigma-Aldrich,
Le fluide interstitiel (ISF) artificiel a été préparé en dissolvant 22 g.L$^{-1}$ de BSA dans un tampon PBS (PBS 1X : 137 mM NaCl, 2.7 mM KCl, 1 mM $Na_2HPO_4$, 1.8 mM $KH_2PO_4$, pH 7.4) et en ajustant le pH à 7,4 si besoin.
Les solutions de glucose dans le PB (0,1M de $Na_2HPO_4$ ; pH = 7,4) et le PBS ont été préparées à 1M en solubilisant la quantité adéquate de glucose dans le PB ou PBS et en ajustant le pH à 7,4 si besoin.

**Exemple 1**

**Préparation de dextrans modifiés chimiquement**

Des dextrans méthacrylés ont été synthétisés selon la méthode décrite dans la demande FR 2 114 492.

Le protocole détaillé est comme suit : du dextran (5 g) a été dissous dans 100 mL d'eau distillée, DW, dans un bécher. Après dissolution complète, différents équivalents d'anhydride méthacrylique de 0,0625 à 0,5 équivalent par rapport aux fonctions hydroxyles du dextran ont été ajoutés goutte à goutte dans la solution de polymère afin d'obtenir le degré souhaité de substitution (DS) du polymère, de 9, 18, 37 ou 62%. Ensuite, le pH a été ajusté à l'aide de NaOH (3 mol.L$^{-1}$) pour être autour de 9 - 11 pendant toute la réaction. La solution a été agitée à température ambiante pendant 1 h. Enfin, le dextran modifié a été dialysé contre de l'eau distillée pendant 1 semaine (membrane 12-14 kDa) et lyophilisé. Le solide blanc a été stocké à -20°C avant utilisation.

**Caractérisations des hydrogels de dextran-méthacylate photo-réticulés**

Une solution de PBS contenant 20 % (p/p) de Dex-MA avec un DS de 9, 18, 37 ou 62 % et 1 % (p/p) de (lithium phenyl-2,4,6-trimethylbenzoylphosphinate) LAP comme photoinitiateur, a été préparée dans un flacon de 10 ml. 100 µL de solution ont été versés dans un moule en Téflon cylindrique de 6 mm de diamètre (sans cavité micro-aiguilles) et la solution a été laissée sécher à l'air pendant 24h. Le polymère a été réticulé par une première irradiation du dos du patch à 405 nm pendant 1 min (P = 75 mW/cm2), suivie du démoulage du cylindre de polymère sec et d'une dernière irradiation à 405 nm pendant 1 min sur l'autre face du cylindre.

Ces 4 matériaux ont été caractérisés par un test de gonflement.

Pour ce faire, les 4 matériaux Dex-MA photo-réticulés séchés ont été pesés (poids WO). Les échantillons ont été placés dans un flacon contenant du PBS (1X) à température ambiante (21-24°C). Ils ont été retirés toutes les heures et leur surface a été rapidement essuyée avec une serviette en papier pour éliminer l'excès d'eau. Ensuite, les échantillons ont été immédiatement pesés (Wt) et replacés dans du PBS pour les points de mesure suivants. Cette opération a été répétée jusqu'à ce qu'aucun autre changement de poids ne soit détecté. Le taux de gonflement (SR) a été calculé selon SR = (Wt - WO) / WO. Trois expériences ont été réalisées en parallèle pour chaque matériau.

Le tableau 1 ci-après rapporte les valeurs moyennes des taux de gonflement ainsi déterminés.

[Tableau 1]

| Echantillon Dex-MA | Taux de gonflement SR (%) |
|---|---|
| DS=62% | 43.9 ± 2.2 |
| DS=37% | 55.2 ± 2.0 |
| DS=18% | 84.2 ± 1.5 |
| DS=9% | 98.0 ± 2.5 |

Le taux de gonflement SR maximum pour chaque formulation a été atteint en environ 1-2 heures. Une augmentation du SR est corrélée avec une diminution du DS.

La dureté de chacun de ces 4 matériaux a également été caractérisée par un test de compression (à l'état sec avant gonflement).

Ces tests de compression ont été réalisés à l'aide d'un texturomètre TAXT.Plus (Stable Micro Systems, UK).

La vitesse de compression a été fixée à 0,5 mm.s$^{-1}$ avec une force de compression maximale allant jusqu'à 50 N. Les valeurs de module de compression, E, ont été calculées comme la pente de la courbe de la force appliquée en fonction du déplacement. Le tableau 2 rend compte de ces valeurs.

[Tableau 2]

| Echantillon | Module de compression E (MPa) |
|---|---|
| Dex-MA DS=62% | 159 ± 7 |
| Dex-MA DS=37% | 167 ± 10 |
| Dex-MA DS=18% | 161 ± 9 |
| Dex-MA DS=9% | 155 ± 7 |

Il n'est pas constaté d'altération des propriétés mécaniques des matériaux. Il est avantageux de noter que les modules de compression des matériaux Dex-MA séchés photo-réticulés sont bien supérieurs à 10 kPa, la valeur seuil pour percer la peau.

## Exemple 2

### Préparation d'un patch électrode-micro-aiguilles, MN.

a) Préparation d'une électrode de travail (WE) en nanotubes de carbone avec des revêtements de médiateur électrochimique (le 1,10-Phenanthroline-5,6-dione (PLQ)) et d'enzyme (la glucose déshydrogénase flavine adénine dinucléotide-dépendante (FADGDH)).

66 mg de MWCNT [Ø = 9.5 nm, 1.5 µm longueur, ≥ 95% pureté, de chez Nanocyl] ont été dispersés dans 66 mL de N,N- diméthylformamide (DMF) dans un flacon de 100 ml, et mis dans un bain-marie sous sonication pendant 1h30. La suspension a été filtrée sur filtre PTFE à l'aide d'une pompe à vide, lavé à l'eau distillée et laissé 2h sous hotte.

Après filtration, le buckypaper (BPE) résultant a été laissé sécher à température ambiante contre un autre filtre PTFE pendant 24h. Le buckypaper a été délicatement détaché du papier filtre et découpé en électrodes individuelles de Ø = 4 ou 6 mm. Ensuite, les BPE MWCNT ont été modifiés par un premier ajout de 20 µl d'une solution de PLQ (5 mM) dans un mélange d'acétone/H$_2$O (rapport volumique de 1 : 1), et laissés sécher pendant 10 minutes. 30 µL d'une solution d'enzyme FADGDH dans du tampon phosphate (PB : 0.1M, pH = 7.4) (10 mg.mL-1) ont été déposés sur la surface de l'électrode modifiée, et laissés sécher quelques heures. Enfin, le contact électrique a été réalisé à l'aide d'un fil métallique fixé à l'arrière de l'électrode avec de la pâte de carbone et laissé sécher pendant 2h. Le dos de l'électrode a été isolé avec de la pâte de silicone et laissé sécher pendant quelques heures avant utilisation.

b) Préparation du moule en poly(diméthylsiloxane), PDMS, pour le patch MN. Un moule maître a été fabriqué en aluminium par micro-fraisage. Le moule en aluminium était composé d'un réseau cylindrique de 7 mm de diamètre avec 5 x 5 (25) micro-aiguilles en forme de crayons avec des hauteurs respectives de 800, 1000 et 1200 µm, une largeur à la base de 400 µm, et une distance bord à bord entre les aiguilles de 400 µm. Un moule inverse a été préparé en coulant un mélange de PDMS et de son agent de durcissement (rapport de 10 : 1) sur le moule principal, sous vide pour éliminer les bulles d'air, puis durci à 100 °C pendant 2 h. Enfin, les moules maître et inverse ont été refroidis sous air pendant quelques heures et le moule PDMS a été délicatement détaché du moule en aluminium.

c) Préparation du patch MN avec intégration de l'électrode de travail Une solution de PBS contenant 20 % (p/p) de Dex-MA avec un DS de 9, 18, 37 ou 62 % (p/p) et 1 % (p/p) de LAP comme photoinitiateur, a été préparée dans un flacon de 10 ml. 100 µl de solution ont été versés dans chaque moule PDMS. Le moule a été placé dans un support en aluminium relié à une pompe à vide, et une pression réduite (1-10 mbar) a été appliquée pendant 2h pour remplir les pointes du moule. L'électrode de travail, WE, préparée en a) a ensuite été intégrée dans le patch MN en insérant l'électrode à l'intérieur de la solution polymère de telle sorte que la surface de l'électrode soit proche de l'arrière des pointes, et l'arrière de l'électrode a été exposé pour permettre la connexion électrique. Le patch MN avec l'électrode, WE, intégrée a ensuite été laissé sécher pendant 24h. Le polymère a été réticulé par une première irradiation du dos du patch à 405 nm pendant 1 min (P = 75 mW/cm2), suivie du démoulage du dispositif et d'une dernière irradiation à 405 nm pendant 1 min sur la face de l'aiguille du patch. Enfin, le raccordement électrique a été effectué en suivant la même procédure que celle décrite ci-dessous.

d) Variante de patch MN avec intégration d'une électrode de référence, RE et/ou une contre-électrode, CE, et une électrode de travail avec une électrode CE et/ou une électrode RE (MN-RE, MN-CE, MN-RE-CE, MN-WE-RE, MN-WE-RE-CE).

Une électrode RE constituée d'un fil d'Ag chloré par chronoampérométrie dans une solution d'HCl à 1M à 2V pendant 5 s (Ag/AgCl) ou une électrode CE constituée d'un fil Pt ont été insérées séparément dans un patch Dex-MA MN avec le même protocole que celui décrit en c).

Une électrode RE constituée d'un fil Ag chloré et une électrode CE constituée d'un fil Pt ont été simultanément insérées dans un patch Dex-MA MN avec le même protocole que celui décrit en c).

Trois électrodes ont été insérées simultanément dans un patch Dex-MA MN : l'électrode BPE décrite en b), une électrode RE constituée d'un fil Ag chloré et une électrode CE constituée d'un fil de Pt. Le dispositif à 3 électrodes intégrées a été préparé comme décrit en a), les 3 électrodes étant insérées sans contact dans la phase polymère visqueuse avant séchage complet et photo-réticulation.

**Exemple 3 :**

**Test de performance en chronoampérométrie d'un dispositif comprenant plusieurs électrodes (MN-WE-CE-RE) intégrées au patch MN en Dex-MA tel qu'obtenu en exemple 2**

La biodétection par mesure chronoampérométrique a été réalisée à température ambiante dans un tampon phosphate (0,1 M, pH = 7,4) à - 0,1 V vs pseudo référence Ag/AgCl à l'aide d'un appareil Princeton Applied research PARSTAT MC (PMC 1000/DC) exécutant le logiciel Versa Studio avec un patch MN de degré de substitution de 37% intégrant 3 électrodes comme décrit dans l'exemple 2d (une électrode RE constituée d'un fil Ag chloré, un fil de platine autonome comme contre-électrode (CE) et l'électrode WE de l'exemple 2a)).

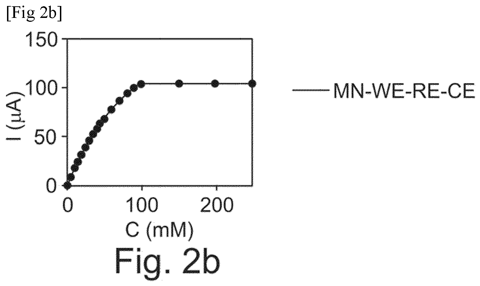

Les mesures de chronoampérométrie sont illustrées en Fig 2a et Fig 2b. La courbe d'étalonnage en Fig 2b est obtenue à partir des mesures de la Fig 2a en prenant les valeurs au plateau du courant obtenues après chaque ajout de glucose. Il est constaté une gamme linéaire d'évolution du courant avec la concentration de glucose sur des concentrations physiologiquement pertinentes.

Les paramètres analytiques sont présentés au Tableau 3.

[Tableau 3]

| Potentiel de travail (V) | Gamme linéaire (mM) ; $R^2$ | Sensibilité ($\mu$A. $mM^{-1}.cm^{-2}$) | Limite de détection LOD ($\mu$M) | Temps de stabilisation (s) |
|---|---|---|---|---|
| -0.1 | 0.3 - 50 ; 0.995 | 1.51 $\pm$ 0.03 | 300 | 83.2 $\pm$ 7.4 |

L'ensemble de ces résultats démontrent la fonctionnalité du capteur intégré.

**Exemple 4** :

**Test de biocompatibilité**

Des cellules de fibroblastes murins NIH-3T3 ont été achetées auprès de l'American Type Culture Collection (ATCC). Les cellules ont été cultivées sous une atmosphère humidifiée (90%) de 95% air/5% CO2 à 37°C, dans du milieu de culture à haute teneur en glucose (milieu Eagle additionné de 10 % de sérum bovin foetal et de 1 % d'antibiotiques (pénicilline et streptomycine) commercialisé par Dubelco (Gibco). Le milieu de culture a été changé tous les deux jours.
Les essais de cytotoxicité ont été réalisés en suivant la norme ISO-10993-5 sur :

1) des échantillons de Dex-MA photo-réticulés à 405 nm pendant 1 min (300 mW/cm2) (obtenus par séchage à air libre et température ambiante de solutions aqueuses de Dex-MA (DS = 37%) et LAP avec des concentrations respectives de 20 et 1%w poids/poids), (matériau hydrogel biocompatible réticulé et gonflant seul) : échantillon MN
2) les électrodes seules c'est-à-dire non intégrées dans un dispositif selon l'invention :

- l'électrode de travail décrite à l'exemple 2 (WE)
- une électrode RE constituée d'un fil Ag chloré
- une contre-électrode faite d'un fil de platine (CE)

3) Des dispositifs selon l'invention préparés selon le protocole détaillé ci-dessus avec une ou plusieurs électrodes intégrées selon les combinaisons qui suivent

- dispositifs intégrant une seule électrode :

  - MN-WE

  - MN-RE

  - MN-CE

- dispositif intégrant 2 électrodes (RE + CE) : MN-RE-CE
- dispositif intégrant 3 électrodes (WE + RE + CE) : MN-WE-RE-CE

Tous les matériaux à tester ont été stérilisés en les plaçant dans une plaque 24 puits, puis en les immergeant entièrement dans un mélange éthanol (70%)/eau (30%) pendant 3h. La solution d'éthanol/eau a ensuite été retirée et remplacée par du PBS stérile pendant 1h afin d'éliminer toute trace d'éthanol. Le PBS stérile a ensuite été remplacé par du PBS stérile frais et laissé pendant 1h, puis du PBS stérile incubé toute une nuit (environ 16h).
Les matériaux ont ensuite été placés dans une nouvelle plaque 24 puits et immergés dans 1 mL de milieu de culture ([C] = 3 cm$^2$ de matériau pour 1 mL de milieu de culture cellulaire) pendant 24h, 48h et 7 jours. Des plaques de contrôle sans matériau avec du milieu de culture cellulaire ont également été réalisées en tant que contrôles négatifs de cytotoxicité. Les milieux de cultures (milieux de relargage) ont ensuite été entièrement collectés après 24h, 48h et 7 jours dans des tubes Eppendorf, puis congelés dans un congélateur à -20°C.
Les cellules ont été ensemencées avec une densité cellulaire de 5000 cellules par puits dans une plaque à 96 puits pendant 24h à 37.5°C et 5% CO2. Après 24h, le milieu de croissance a été remplacé par 100 $\mu$L de milieu de relargage (décongelé à 37,5°C) ou de milieu de contrôle (contrôle négatif) ou de milieu de culture cellulaire contenant 10 mM de H2O2 (contrôle positif de cytotoxicité). Après 24 h, les milieux de culture cellulaires ont été retirés et remplacés par 100 $\mu$L de milieu de culture cellulaire frais et 10 $\mu$L de réactif de prolifération cellulaire (WST-1 de Roche Diagnostics GmbH) ont été ajoutés. Après 2 h d'incubation à 37,5°C et 5% CO2, la viabilité cellulaire a été calculée en lisant l'absorbance à 450 nm corrigée de l'absorbance à 650 nm avec un lecteur de microplaques Infinite-M1000 TECAN suivant la formule suivante :

$$Viabilité~(\%) = \frac{Absorbance_{\text{échantillon}} - Absorbance_{\text{contrôle positif}}}{Absorbance_{\text{controle négatif}} - Absorbance_{\text{contrôle positif}}} * 100$$

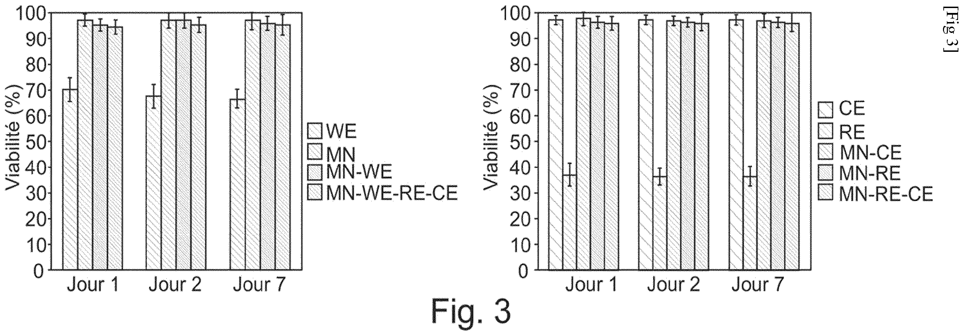

Les expériences ont été réalisées sur 5 échantillons différents et sur des lots de cellules différents et à des temps différents. Les résultats sont présentés en Fig 3.

Les essais de cytotoxicité sur les échantillons montrent une bonne cytocompatibilité des micro-aiguilles à 20% p/p de Dex-MA de DS = 37% réticulé avec 1%p/p de LAP (MN) avec une viabilité cellulaire supérieure à 80% (94,8% à 96%), et une bonne biocompatibilité de tous les dispositifs suivant le principe de l'invention.

Par contraste, les électrodes non-intégrées dans le patch MN et le dispositif présentent de la cytotoxicité, avec des viabilités comprises entre 67,2 et 71,8% pour la WE, et entre 36.1 et 36.9% pour la RE.

## Exemple 5

### Test de stabilité d'une électrode de travail collée au dos d'un patch MN

Une solution de PBS contenant 20 % (p/p) de Dex-MA avec un DS de 37% et 1 % (p/p) de LAP comme photoinitiateur, a été préparée dans un flacon de 10 ml. 100 $\mu$l de solution ont été versés dans le moule PDMS. Le moule a été placé dans un support en aluminium relié à une pompe à vide, et une pression réduite (1-10 mbar) a été appliquée pendant 24h pour remplir les pointes du moule et sécher le polymère. Le polymère sec a ensuite été réticulé sous UV-vis à 405 nm pendant 1 min (P = 75 mW/cm2). Le biocapteur BPE (préparé en 2a)) a ensuite été intégré en déposant une couche de solution de polymère contenant 20 % (p/p) de Dex-MA avec un DS de 37% et 1% (p/p) de LAP sur le biocapteur et en collant l'électrode au dos du patch. Le patch MNs a ensuite été exposé de nouveau aux UV-vis à 405 nm pendant 1 min avant de réticuler la couche collante. Enfin, le raccordement électrique a été effectué en suivant la même procédure que celle décrite ci-dessous.

Le dispositif a ensuite été analysé par chronoampérométrie à -0,1V dans une cellule à 3 électrodes employant une électrode de platine comme CE, un fil Ag chloré comme RE, le biocapteur comme WE et en immergeant la partie hydrogel du biocapteur dans une solution de PB sous agitation pendant 1h, suivi par un ajout de 5 mM de glucose. Toutefois, par opposition au patch MN selon l'invention et testé en exemple 3, aucun courant catalytique n'a été observé après 24h. Un décollement partiel de l'électrode a également été observé.

## Revendications

1. Dispositif comportant au moins un support polymérique (1) ayant une surface (4) dédiée à un contact avec une peau (2), au moins un réseau de micro-aiguilles polymériques pleines (3) solidaires dudit support (1) et dépassant vers l'extérieur de ladite surface (4) du support dédiée au contact avec ladite peau (2) et au moins une électrode (9) **caractérisé en ce que** lesdites micro-aiguilles (3) et au moins la surface (4) de contact dudit support (1) avec ladite peau (2) sont formées d'un hydrogel réticulé, non conducteur électronique à l'état sec et conducteur électrolytique au contact d'un fluide aqueux, et **en ce que** la, une, des ou lesdites électrodes (9) est (sont) disposées au contact de l'hydrogel dédié à gonfler au contact d'un fluide aqueux, est (sont) dénuées de contact direct avec la peau (2) et est (sont) intégrée(s) dans l'hydrogel réticulé constitutif du support (1) qui est en contact direct avec l'arrière des micro-aiguilles (3) et/ou intégrées dans l'hydrogel réticulé constitutif de microaiguille(s) (3).

2. Dispositif selon la revendication précédente dans lequel l'ensemble support (1) et micro-aiguilles (3) est constitué dudit hydrogel réticulé, non conducteur et gonflant au contact d'un fluide aqueux.

3. Dispositif selon l'une quelconque des revendications précédentes **caractérisé en ce que** ledit hydrogel est dénué de constituant métallique et de polymère conducteur électronique.

4. Dispositif selon l'une quelconque des revendications précédentes dans lequel ledit hydrogel est obtenu par réticulation d'un ou plusieurs polymères synthétiques, d'un ou plusieurs biopolymères, le cas échéant modifiés chimiquement pour être réticulables, ou d'un de leurs mélanges.

5. Dispositif selon la revendication précédente dans lequel ledit biopolymère est choisi parmi les polyhydroxyacides, tels que par exemple l'acide polyglycolique (PGA) et l'acide polylactique (PLA), également connu sous le nom de polylactide ; les polysaccharides, tels que par exemple la cellulose, la chitine et l'amidon et des protéines ou des peptides, tels que par exemple le collagène, la gélatine, les élastines, les fibroïnes, la zéine de maïs, la soie de diverses espèces de vers à soie et la kératine et leurs dérivés.

6. Dispositif selon la revendication précédente dans lequel ledit biopolymère est ou dérive d'au moins un polymère choisi parmi les alginates, l'acide hyaluronique, la carboxyméthylcellulose, le chitosan et le dextran et leurs dérivés et, de préférence, le dextran.

7. Dispositif selon l'une quelconque des revendications précédentes dans lequel ledit hydrogel dérive de la réticulation d'au moins un polymère de dextran modifié par des motifs choisis parmi les acrylate, méthacrylate, alcényle et alcynyle et en particulier un polymère de dextran modifié par des motifs méthacrylate, DexMA, et de DS variant de 5 à 65 %.

8. Dispositif selon l'une quelconque des revendications précédentes, dont les micro-aiguilles (3) ont une longueur variant de 200 à 3000 $\mu$m.

9. Dispositif selon l'une quelconque des revendications précédentes, comportant une ou plusieurs électrodes choisies parmi des électrodes de référence, contre-électrodes, électrodes de travail telles qu'électrodes sélectives aux ions (ISE), électrodes de pH, électrodes électrocatalytique, bioélectrodes, électrodes micro ou nanostructurées.

10. Dispositif selon l'une quelconque des revendications précédentes comportant une ou plusieurs électrodes 9 choisie parmi les électrodes ou bioélectrodes à base de Pt, Au, Ag, Pd, Ni, Ir, carbone graphitique, carbone amorphe, graphène, oxyde de graphène, diamant, diamant dopé au bore, nanotubes, fibres dopées semi-conductrices, de préférence silicium dopé, oxydes métalliques, de préférence oxyde d'indium-étain, et les électrodes polymères conducteurs, de préférence PEDOT et fibres conductrices.

11. Dispositif selon l'une quelconque des revendications précédentes, comportant au moins deux voire d'au moins trois voire quatre électrodes (9).

12. Dispositif selon l'une quelconque des revendications précédentes se présentant sous la forme d'un patch micro-aiguilles, MN.

13. Dispositif selon l'une quelconque des revendications 1 à 12 comportant un dispositif de détection électrochimique attaché à ladite ou aux dites électrodes (9) via des connections électriques.

14. Procédé de préparation d'un dispositif selon l'une quelconque des revendications précédentes par micromoulage et comprenant au moins la réticulation d'au moins un polymère pour former ledit hydrogel réticulé **caractérisé en ce que** la ou les électrodes (9) dudit dispositif sont intégrées par mise en contact avec ledit polymère avant ou simultanément à sa réticulation.

15. Procédé selon la revendication précédente dans lequel ledit polymère est mise en oeuvre sous la forme d'une formulation aqueuse liquide ou semi-liquide et, de préférence, présentant une viscosité variant de 1 à 100 Pa.s$^{-1}$ à 1 Hz lorsque la contrainte de cisaillement appliquée est de 0,2 %.

16. Procédé selon la revendication précédente dans lequel la ou les électrodes (9) sont disposées en surface de ladite formulation aqueuse contenant au moins ledit polymère non réticulé et y subissent une pression mécanique pour les positionner au sein de l'hydrogel constitutif de micro-aiguilles (3) et/ou en profondeur à proximité de la base des micro-aiguilles (3) préalablement ou simultanément à la réticulation.

17. Procédé selon la revendication 14, 15 ou 16 dans lequel la réticulation est réalisée par voie photochimique.

18. Procédé selon l'une quelconque des revendications 14 à 17 dans lequel ledit hydrogel est tel que défini en revendications 5 à 7.

19. Utilisation d'un dispositif selon l'une quelconque des revendications 1 à 13 pour la stimulation électrique.

20. Utilisation d'un dispositif selon l'une quelconque des revendications 1 à 13 pour la génération d'énergie et notamment à titre de pile enzymatique à biocombustible.

21. Utilisation d'un dispositif selon l'une quelconque des revendications 1 à 13 pour le stockage d'énergie et notamment à titre d'un supercondensateur ou d'un dispositif hybride de pile enzymatique à biocombustible-supercondensateur.

22. Utilisation d'un dispositif selon l'une quelconque des revendications 1 à 13 pour des mesures électrochimiques transdermiques, en particulier pour la caractérisation d'au moins un analyte dans un liquide interstitiel (6).

[Fig 1]

Fig. 1

[Fig 2a]

Fig. 2a

[Fig 2b]

Fig. 2b

[Fig 3]

EP 4 487 772 A1

Fig. 3

EP 4 487 772 A1

Europäisches
Patentamt
European
Patent Office
Office européen
des brevets

# RAPPORT DE RECHERCHE EUROPEENNE

Numéro de la demande

EP 24 18 6522

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | MENGJIA ZHENG ET AL: "Osmosis-Powered Hydrogel Microneedles for Microliters of Skin Interstitial Fluid Extraction within Minutes", ADVANCED HEALTHCARE MATERIALS, WILEY - V C H VERLAG GMBH & CO. KGAA, DE, vol. 9, no. 10, 30 avril 2020 (2020-04-30) , page n/a, XP072469317, ISSN: 2192-2640, DOI: 10.1002/ADHM.201901683 | 1-13, 19-22 | INV. A61B5/145 A61B5/00 |
| Y | * abrégé * <br> * page 2 * <br> * pages 6-9 * <br> * figures 1,6 * | 14-18 | |
| Y | WO 2022/040177 A1 (UNIV CALIFORNIA [US]) 24 février 2022 (2022-02-24) * alinéa [0052]; figure 4B * | 14-18 | |
| X | CALIÒ A ET AL: "Polymeric microneedles based enzymatic electrodes for electrochemical biosensing of glucose and lactic acid", SENSORS AND ACTUATORS B: CHEMICAL, ELSEVIER BV, NL, vol. 236, 1 juin 2016 (2016-06-01), pages 343-349, XP029700070, ISSN: 0925-4005, DOI: 10.1016/J.SNB.2016.05.156 * 2.Experimental; pages 344-345 * * figures 1-4 * | 1-4, 8-13,22 | DOMAINES TECHNIQUES RECHERCHES (IPC) A61B |

-/--

Le présent rapport a été établi pour toutes les revendications

| Lieu de la recherche | Date d'achèvement de la recherche | Examinateur |
|---|---|---|
| La Haye | 13 septembre 2024 | Bataille, Frédéric |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 24 18 6522

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | MOHAN A M VINU ET AL: "Continuous minimally-invasive alcohol monitoring using microneedle sensor arrays", BIOSENSORS AND BIOELECTRONICS, ELSEVIER SCIENCE LTD, UK, AMSTERDAM , NL, vol. 91, 10 janvier 2017 (2017-01-10), pages 574-579, XP029920725, ISSN: 0956-5663, DOI: 10.1016/J.BIOS.2017.01.016 * section 2.2; pages 575-576 * * section 4 * * figures * | 1-22 | |
| A | EP 4 201 966 A1 (COMMISSARIAT ENERGIE ATOMIQUE [FR]; CENTRE NAT RECH SCIENT [FR] ET AL.) 28 juin 2023 (2023-06-28) * alinéas [0006] - [0008]; revendications * | 7 | |
| A | US 2011/257504 A1 (HENDRICKS JEFFREY L [US] ET AL) 20 octobre 2011 (2011-10-20) * alinéas [0003], [0065] * | 19 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| La Haye | 13 septembre 2024 | Bataille, Frédéric |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

                                                                 
& : membre de la même famille, document correspondant

EPO FORM 1503 03.82 (P04C02)

**EP 4 487 772 A1**

## ANNEXE AU RAPPORT DE RECHERCHE EUROPEENNE
## RELATIF A LA DEMANDE DE BREVET EUROPEEN NO.

EP 24 18 6522

La présente annexe indique les membres de la famille de brevets relatifs aux documents  brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

13-09-2024

| Document brevet cité au rapport de recherche | Date de publication | Membre(s) de la famille de brevet(s) | Date de publication |
|---|---|---|---|
| WO 2022040177 A1 | 24-02-2022 | US 2023277080 A1<br>WO 2022040177 A1 | 07-09-2023<br>24-02-2022 |
| EP 4201966 A1 | 28-06-2023 | EP 4201966 A1<br>FR 3131316 A1 | 28-06-2023<br>30-06-2023 |
| US 2011257504 A1 | 20-10-2011 | AUCUN | |

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20060264716 A
- US 20090099427 A
- US 20140336487 A
- FR 2114492

**Littérature non-brevet citée dans la description**

- **SANJIV SHARMA**. *Anal Bioanal Chem*, 2016, vol. 408, 8427-8435
- **FARSHAD TEHRANI**. *Nature Biomédical Engineering*, 2022
- **TEYMOURIAN**. *Anal. Chem.*, 2020, vol. 92 (2), 2291-2300
- **AM VINU MOHAN**. *Biosensors andBioelectronics*, 2017, vol. 91, 574-5791
- **PEYMAN G**. *Adv Health Mater.*, 2022